# EUROPÄISCHE PATENTSCHRIFT

(11) **EP 0 567 846 B1**
(45) Veröffentlichungstag und Bekanntmachung des Hinweises auf die Patenterteilung: **04.12.1996**
(21) Anmeldenummer: 93106053.7
(22) Anmeldetag: 14.04.1993
(51) Int. Cl.: C09B 26/06, C09B 56/20, C07D 285/135

(54) **Kationische Triazatrimethinfarbstoffe**
Cationic triazatrimethine dyes
Colorants cationiques de type triazatriméthine

(30) Priorität: 27.04.1992 US 874674; 15.12.1992 DE 4242429
(43) Veröffentlichungstag der Anmeldung: 03.11.1993
(73) Patentinhaber: BAYER AG, 51368 Leverkusen (DE)
(72) Erfinder: Fürstenwerth, Hauke, Dr., W-5090 Leverkusen 3 (DE); Lange, Karl-Heinrich, Dr., W-5093 Burscheid 2 (DE); Raue, Roderich, Dr., W-5090 Leverkusen (DE); Brack, Alfred, Dr., W-5068 Odenthal (DE)

(56) Entgegenhaltungen:
- EP-A- 0 053 751
- EP-A- 0 446 731
- BE-A- 572 834
- DE-B- 1 069 563
- GB-A- 2 017 134

## Beschreibung

Die vorliegende Erfindung betrifft neue kationische Triazatrimethinfarbstoffe, Verfahren zu deren Herstellung und deren Verwendung zum Färben und Bedrucken natürlicher und synthetischer Materialien insbesondere von Fasern aus Polyacrylnitril und sauer modifizierten Polyestern und Polyamiden.

Die neuen Triazatrimethinfarbstoffe entsprechen der allgemeinen Formel (I) 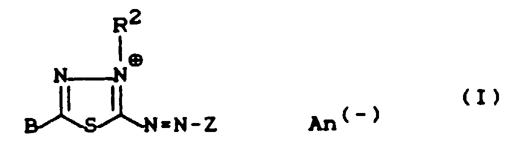 worin
- Z: für einen Rest der Formel 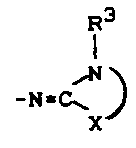 steht, worin
- R³: für Wasserstoff, einen Alkyl-, Alkenyl- oder Aralkylrest steht, und
- X: für die restlichen Glieder eines fünfgliedrigen, gegebenenfalls substituierten und gegebenenfalls anellierten partiell ungesättigten Heterocyclus, der ein bis drei Heteroatome aus der Reihe Sauerstoff, Schwefel, Stickstoff enthält, steht
- B: für Alkylmercapto, Arylmercapto oder einen Rest der Formel 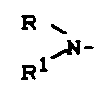
worin
- R: Wasserstoff, einen Alkyl-, Alkenyl-, Acyl-, Cycloalkyl-, Aryl-, Aralkyl-, Amino-, Alkylamino-, Dialkylamino-, Arylamino-, Aralkylamino- oder heterocyclischen Rest und
- R¹: Wasserstoff, einen Alkyl-, Alkenyl- oder Aralkylrest bedeuten, oder
- R und R¹: gemeinsam mit dem Stickstoffatom, an das sie gebunden sind, einen Heterocyclus bilden,
- R₂: für Wasserstoff, einen Alkyl-, Alkenyl-, Alkinyl- oder Aralkylrest steht,
und
- An⁽⁻⁾: für ein Anion steht
und worin die cyclischen und acyclischen Reste nicht ionogene Substituenten und/oder eine Carboxylgruppe enthalten können.

Nichtionogene Substituenten im Sinne der vorliegenden Erfindung sind die in der Farbstoffchemie üblichen nicht dissoziierenden Substituenten, wie z.B. Cyan, Hydroxy, Halogen, wie Fluor, Chlor oder Brom, Nitro, Alkyl, Mono-und Dialkylamino, Phenyl, Alkoxy, Acyloxy, Alkoxycarbonyl, Alkoxycarbonyloxy, wobei Allyl und Alkoxy vorzugsweise 1 bis 4 C-Atome enthalten und Acyl insbesondere für C₁-C₄-Alkylcarbonyl steht.

Im Rahmen dieser Erfindung sind Alkylreste auch solche in zusammengesetzten Begriffen wie Alkylamino beispielsweise solche mit 1 bis 8, insbesondere 1 bis 4 C-Atomen.

Substituenten der Alkylreste R¹, R² und R³ sind beispielsweise Halogen, Hydroxy, C₁-C₄-Alkoxy, Phenyloxy, Benzyloxy, C₁-C₄-Alkoxycarbonyl, Carboxyl, Amidocarbonyl oder Cyan, Halogen steht vorzugsweie für Fluor, Chlor und Brom. Unter Alkenyl- und Alkinylresten werden insbesondere solche mit 2 bis 5 C-Atomen verstanden.

Cycloalkyl steht z.B. für gegebenenfalls durch C₁-C₄-Alkyl substituiertes Cyclopentyl oder Cyclohexyl. Geeignete Acylreste sind z.B. C₁-C₃-Alkylcarbonyl, gegebenenfalls durch Halogen oder C₁-C₄-Alkyl substituiertes Benzoyl, C₁-C₄-Alkoxycarbonyl, Mono- und Di-C₁-C₄-alkylaminocarbonyl, Benzylaminocarbonyl, C₁-C₄-Alkylsulfonyl, Phenylsulfonyl oder Di-C₁-C₄-alkylaminosulfonyl.

Unter Aryl wird im allgemeinen Phenyl oder Naphthyl und unter Aralkyl Benzyl und β-Phenyl-C₂-C₄-alkyl verstanden. Die Phenylreste können durch beispielsweise 1 bis 3 nichtionogene Reste wie Halogen, C₁-C₄-Alkyl, C₁-C₄-Alkoxy, C₁-C₄-Alkoxycarbonyl, Carboxyl, Amidocarbonyl, Cyan, Nitro, Amidosulfonyl, C₁-C₃-Alkylcarbonylamino, Benzoylamino, Hetaryl oder Arylazo substituiert sein. Unter Hetaryl wird dabei z.B. gegebenenfalls durch C₁-C₄-Alkyl oder C₁-C₄-Alkoxy substituiertes Benzthiazolyl verstanden.

Die Reste R und R¹ können zusammen mit dem Stickstoffatom, an das sie gebunden sind, vorzugsweise einen 5- oder 6-gliedrigen Ring wie Pyrrolidin, Piperidin, Morpholin, Piperazin, N-Methyl- oder N-Hydroxyethylpiperazin bilden.

Als anionische Reste kommen die für kationische Farbstoffe üblichen organischen und anorganischen Anionen in Betracht.

Bevorzugt sind farblose Anionen. Das Anion ist im allgemeinen durch das Herstellungsverfahren und die eventuell vorgenommene Reinigung des rohen Farbstoffes gegeben. Im allgemeinen liegen die Farbstoffe als Halogenide (insbesondere als Chloride oder Bromide) oder als Methosulfate, Äthosulfate, Sulfate, Benzol- oder Toluolsulfonate oder als Acetate vor. Die Anionen können in bekannter Weise gegen andere Anionen ausgetauscht werden.

Bevorzugt sind Triazatrimethinfarbstoffe der Formel (I), worin
- Z: für einen Rest der Formel 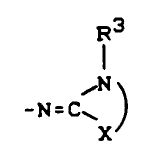
steht, worin
- R³: für Wasserstoff, C₁-C₈-Alkyl, welches gegebenenfalls durch Hydroxy, Halogen, Cyano, C₁-C₄-Alkoxy, Carboxyl, C₁-C₄-(Di)-alkylamino, C₁-C₄-Alkoxycarbonyl oder C₁-C₄-Alkylsulfonyl substituiert ist, für C₂-C₄-Alkenyl oder für Phenyl-C₁-C₂-alkyl, welches gegebenenfalls durch Halogen, Cyano, C₁-C₄-Alkyl oder C₁-C₄-Alkoxy substituiert ist, und
- X: für die restlichen Glieder eines Thiazolin-, Isothiazolin-, Benzothiazolin-, 1,2,4-Triazolin-, 1,3,4-Thiadiazolin-, Oxazolin-, Benzoxazolin-, Imidazolin-, Benzimidazolin-, Pyrazolin- oder Benzpyrazolinrings, welcher jeweils gegebenenfalls ein- oder zweifach, gleich oder verschieden substituiert ist, durch C₁-C₈-Alkyl, welches gegebenenfalls durch Hydroxy, Halogen, Cyano, C₁-C₄-Alkoxy, Carboxyl, C₁-C₄-(Di)alkylamino, C₁-C₄-Alkoxycarbonyl oder C₁-C₄-Alkylsulfonyl substituiert ist, für C₂-C₄-Alkenyl oder für Phenyl-C₁-C₂-alkyl, welches gegebenenfalls durch Halogen, Cyano, C₁-C₄-Alkyl oder C₁-C₄-Alkoxy substituiert ist,
- B: für einen Rest der Formel 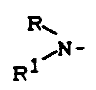
steht, worin
- R: für Wasserstoff, einen gegebenenfalls durch Hydroxy, Halogen, Cyan, C₁-C₄-Alkoxy, Carboxyl, Aminocarbonyl oder C₁-C₄-Alkoxycarbonyl substituierten C₁-C₈-Alkylrest, einen C₂-C₄-Alkenylrest, einen gegebenenfalls durch C₁-C₄-Alkyl substituierten Cyclohexylrest, einen gegebenenfalls durch Halogen, C₁-C₄-Alkyl oder C₁-C₄-Alkoxy substituierten Phenyl-, Benzyl- oder Phenylethylrest, einen C₁-C₄-Alkyloxycarbonyl-, Mono- oder Di-C₁-C₄-alkylaminocarbonyl-, Aminocarbonyl-, Mono-oder Di-C₁-C₄-alkylaminosulfonyl-, Amino-, C₁-C₄-Alkylamino-, Di-(C₁-C₄-alkyl)-amino-, einen gegebenenfalls durch C₁-C₄-Alkyl oder C₁-C₄-Alkoxy substituierten Phenylamino- oder Benzylamino-Rest und
- R¹: für Wasserstoff, einen gegebenenfalls durch Hydroxy, Halogen, Cyan, C₁-C₄-Alkoxy, Carboxyl, Aminocarbonyl oder C₁-C₄-Alkoxycarbonyl substituierten C₁-C₈-Alkylrest, einen C₂-C₄-Alkenylrest oder einen gegebenenfalls durch Halogen, C₁-C₄-Alkyl oder C₁-C₄-Alkoxy substituierten Phenyl-, Benzyl- oder Phenylethylrest steht
und
- R²: für Wasserstoff, einen gegebenenfalls durch Hydroxy, Halogen, Cyan, C₁-C₄-Alkoxy, Carboxyl, Aminocarbonyl oder C₁-C₄-Alkoxycarbonyl substituierten C₁-C₈-Alkylrest, einen C₂-C₄-Alkenylrest oder einen gegebenenfalls durch Halogen, C₁-C₄-Alkyl oder C₁-C₄-Alkoxy substituierten Benzyl-oder Phenylethylrest steht.

Besonders bevorzugt sind Farbstoffe der Formel (I) worin
- Z: für einen Rest der Formel 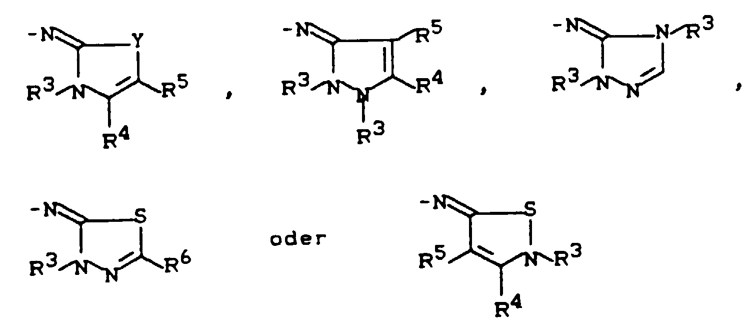
steht worin
- Y: für -S-, -O- oder 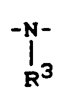 steht,
wobei R³ die oben angegebene Bedeutung hat, und beim Auftreten von zwei Substituenten R³ diese gleich oder verschieden sein können,
- R⁴ und R⁵: entweder für Wasserstoff stehen, oder gemeinsam für die restlichen Glieder eines anellierten Benzolrings stehen, und
- R⁶: für C₁-C₄-(Di)alkylamino, C₆-C₁₀-(Di)arylamino, Piperazino, Pyrrolidino, C₁-C₄-Alkylmercapto oder C₆-C₁₀-Arylmercapto steht,
- B: für einen Rest der Formel 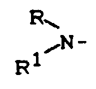 steht,
worin
- R: für Wasserstoff, einen gegebenenfalls durch Hydroxy, Halogen, Cyan, C₁-C₄-Alkoxy, Carbonyl, Aminocarbonyl oder C₁-C₄-Alkoxycmrbonyl substituierten C₁-C₄-Alkylrest, einen gegebenenfalls durch Halogen, C₁-C₄-Alkyl oder C₁- C₄-Alkoxy substituierten Phenyl-, Benzyl-oder Phenylethylrest,
- R¹: für einen gegebenenfalls durch Hydroxy, Halogen, Cyan, C₁-C₄-Alkoxy, Carboxy, Aminocarbonyl oder C₁-C₄-Alkoxycarbonyl substituierten C₁-C₄-Alkylrest oder einen gegebenenfalls durch Halogen, C₁-C₄-Alkyl oder C₁-C₄-Alkoxy substituierten Benzyl- oder Phenylethylrest steht
und
- R²: für Wasserstoff, einen gegebenenfalls durch Hydroxy, Halogen, Cyan, C₁-C₄-Alkoxy, Carboxy, Aminocarbonyl oder C₁-C₄-Alkoxycarbonyl substituierten C₁-C₈-Alkylrest oder einen gegebenenfalls durch Halogen, C₁-C₄-Alkyl oder C₁-C ₄-Alkoxy substituierten Benzyl- oder Phenylethylrest steht.

Die neuen Triazatrimethinfarbstoffe der Formel (I) können in bekannter Weise hergestellt werden, durch Umsetzung von Farbbasen der Formel (II) 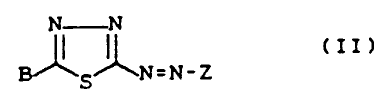 worin
B und Z die oben angegebenen Bedeutungen haben mit einem Alkylierungsmittel aus der Klasse der Alkylhalogenide, Michael-Akzeptoren oder Epoxide, wie sie beispielsweise auch in DE-A 2 811 258 auf Seite 34 genannt sind.

Für den Fall, daß der Substituent B in Formel (II) sterisch wenig anspruchsvoll ist, kann bei der Umsetzung mit dem Quaternierungsmittel auch zu einem gewissen Prozentsatz, der meist weniger als 10 % beträgt, Quaternierung in 4-Stellung des Thiadiazolrings erfolgen.

Die Farbbasen der Formel (II) sind ebenfalls neu und Gegenstand der vorliegenden Erfindung. Die Farbbasen der Formel (II) stellen ebenfalls Farbstoffe dar, deren Verwendung zum Färben von Fasern und Geweben aus mit nichtionischen Farbstoffen anfärbbaren Materialien, z.B. Polyester, sowie von Kunststoffen wie z.B. Polystyrol, Polycarbonat, Polyamid oder Acrylnitril-Butadien-Styrol-Copolymerisaten einen weiteren Erfindungsgegenstand darstellt.

Die Farbbasen der Formel (II) können in an sich bekannter Art und Weise zum Beispiel nach dem in der DE-A 1 069 563 beschriebenen Verfahren durch Diazotierung von heterocyclischen Aminen und Kupplung auf Quartärsalze der gleichen oder unterschiedlicher heterocyclischer Amine hergestellt werden. Die Diazotierung erfolgt in wäßrig mineralsaurer Lösung oder in wäßriger Lösung einer organischen Säure mit Alkalinitriten. Die so erhaltene Diazoniumsalzlösung wird mit der Lösung der Kupplungskomponente vereinigt und das Produkt, gegebenenfalls nach Abpuffern, z.B. durch Filtration isoliert.

Alternativ können die neuen Farbstoffe der Formel (II) durch ein neues Verfahren hergestellt werden, das ebenfalls Gegenstand der vorliegenden Erfindung ist. Das neue Verfahren zur Herstellung von Farbstoffen der Formel (II) ist dadurch gekennzeichnet, daß eine Verbindung der Formel (IV) 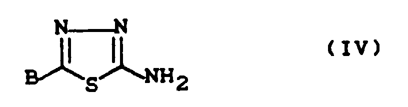 worin
- B: die oben angegebene Bedeutung hat,
und eine Verbindung der Formel (V)

H-Z¹ (V)

worin
- Z¹: für einen Rest der Formel 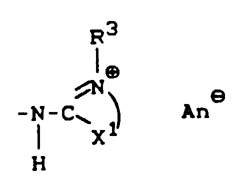
steht, worin
- R³und An^{⊖}: die oben angegebene Bedeutung haben und
- X¹: für die restlichen Glieder eines quaternierten fünfgliedrigen gegebenenfalls substituierten und gegebenenfalls anellierten, ungesättigten Heterocyclus, der ein bis drei Heteroatome aus der Reihe Sauerstoff, Schwefel, Stickstoff enthält, vorzugsweise für die restlichen Glieder eines quaternierten Thiazol-, Isothiazol-, Benzothiazol-, 1,2,4-Triazol-, 1,3,4-Thiadiazol-, Oxazol-, Benzoxazol-, Imidazol-, Benzimidazol-, Pyrazol- oder Benzpyrazolrings steht, welcher jeweils gegebenenfalls ein- oder zweifach, gleich oder verschieden substituiert ist, durch C₁-C₈-Alkyl, welches gegebenenfalls durch Hydroxy, Halogen, Cyano, C₁-C₄-Alkoxy, Carboxyl, C₁-C₄-(Di)alkylamino, C₁-C₄-Alkoxycarbonyl oder C₁-C₄-Alkylsulfonyl substituiert ist, für C₂-C₄-Alkenyl oder für Phenyl-C₁-C₂-alkyl, welches gegebenenfalls durch Halogen, Cyano, C₁-C₄-Alkyl oder C₁-C₄-Alkoxy substituiert ist, steht,
in wäßrigem Medium mit einer salpetrige Säure abgebenden Substanz in Gegenwart von CO₂ unter einem Druck von 5 bis 100 bar bei einer Temperatur von 0 bis 125°C umgesetzt werden. Die Reaktion ist im allgemeinen nach 10

Minuten bis 24 Stunden beendet. Vorzugsweise wird das erfindungsgemäße Verfahren bei einer Temperatur von 0 bis 100°C, besonders bevorzugt von 0 bis 70°C und insbesondere von 30 bis 40°C, durchgeführt.

Als Reaktionsmedium eignet sich Wasser, gegebenenfalls in Mischung mit organischen Lösungsmitteln, die ganz oder teilweise mit Wasser mischbar sind.

Als geeignete Lösungsmittel kommen Methanol, Ethanol, Propanol, Isopropanol, Isoamylalkohol, Ethylenglykol, Methylglykol, Ethylglykol, Butylglykol, Ethylenglykoldimethylether, Ethylenglykoldiethylether, Diethylenglykoldimethylether, Glykoldiacetat, Methylglykolacetat, Ethylglykolacetat, Butylglykolacetat, Propylenglykol, Propylenglykolmonomethylether, Propylenglykolmonoethylether, Propylenglykoldimethylether, Propylenglykoldiethylether, Propylenglykolacetat, Triacetin, Acetonitril, Tetrahydrofuran, Dioxan, Dimethylformamid und N-Methyl-pyrrolidon in Frage.

Bevorzugt ist die Verwendung von Wasser oder einem Gemisch aus Wasser und Methanol, das ein Verhältnis von Wasser zu Methanol von vorzugsweise 1:0,2 bis 1:10, besonders bevorzugt von 1:1 bis 1:5 und insbesondere von 1:2 aufweist.

Bei Normaldruck findet eine Reaktion mit CO₂ nicht statt. Vorzugsweise wird die Reaktion bei einem Druck von 25 bis 65 bar durchgeführt.

Als salpetrige Säure abgebende Substanzen sind insbesondere anorganische und organische Nitrite geeignet.
Von den anorganischen Nitritenkommen vorzugsweise die Salpetrigsäuresalze der Elemente der ersten, zweiten und dritten Hauptgruppe des Periodensystems sowie der Übergangsmetalle in Frage, wie z.B. Lithiumnitrit, Natriumnitrit, Kaliumnitrit, Magnesiumnitrit, Calciumnitrit, Aluminiumnitrit, Eisennitrit, oder Kupfernitrit.

Von den organischen Nitriten sind die Salpetrigsäureester der Formel

E-O-N=O,

worin
- E: für einen gegebenfalls durch OH, -OAlkyl, -OAcetyl oder -ONO substituierten C₁-C₁₀-Alkylrest steht,
bevorzugt. Beispielhaft seien die folgenden organischen Nitrite genannt:

Methylnitrit, Ethylnitrit, n-Propylnitrit, i-Propylnitrit, n-Butylnitrit, i-Butylnitrit, s-Butylnitrit, n-Pentylnitrit, i-Pentylnitrit, alle Isomeren der Hexyl-, Heptyl- und Octylnitrit; 2-Methoxy-ethythylnitrit, 2-Ethoxyethylnitrit, 2-Propoxyethylnitrit, 2-Butoxyethylnitrit, 1-Methoxyprop-2-ylnitrit; weiterhin Salpetrigsäure Mono- und Diester von 1,2-Ethandiol, 1,2-Propandiol, 1,3-Propandiol, 1,4-Butandiol, 1,5-Pentandiol, 1,6-Hexandiol, 2-Methyl-1,3-propandiol, 2,2-Dimethyl- 1,3-propandiol, 2,2-Diethyl- 1,3-propandiol, 1-Propyl-2-ethyl-1,3-propandiol, 1-Propyl-2,2-dimethyl-1,3-propandiol, 2-Ethyl-2-butyl- 1,3-propandiol; Diethylenglykol, Di-1,2-propylenglykol und Triethylenglykol sowie der Methyl-, Ethyl-, Propyl-und Butylhalbether von Diethylenglykol, Di-1,2-Propylenglykol und Triethylenglykol, sowie die Salpetrigsäureester des Pentaerythrits und der daraus abgeleiteten Alkylether.

Die Herstellung von Triazatrimethinfarbstoffen wurde bisher durch Diazotieren der aromatischen oder heterocyclischen Amine in mineralsaurer Lösung, Zugabe der Diazoniumsalzlösung zur Lösung der Kupplungskomponente und Kuppeln durch Zugabe von säurebindenden Mitteln durchgeführt (vgl. z.B. DE-A 1 069 563).

In jedem Fall wurde bisher eine anorganische oder organische Säure zur Diazotierung eingesetzt, die bei der anschließenden Kupplung neutralisiert werden muß. Hierbei entstehen anorganische Salze. Diese anorganischen Salze gelangen bei der Isolierung des Farbstoffes in das Abwasser und stellen eine Belastung dar. In Mischung mit dem hergestellten Farbstoff beeinflussen anorganische Salze häufig die Löslichkeit in nachteiliger Weise. Zur Herstellung stabiler Farbstofflösungen müssen diese Salze durch aufwendige Verfahren, beispielsweise Druckpermeation oder Reverseosmose entfernt werden. Alle diese Nachteile können durch das neue erfindungsgemäße Herstellungsverfahren vermieden werden.

Die erfindungsgemäßen Farbstoffe der Formel (I) eignen sich insbesondere zum Färben und Bedrucken von natürlichen und synthetischen Materialien, insbesondere von Fasern aus Polyacrylnitril, sauer modifizierten Polyestern und Polyamiden.

Das Färben kann aus schwach saurer Flotte erfolgen, wobei man in das Färbebad zweckmäßigerweise bei 40 bis 60⁰C eingeht und dann bei Kochtemperatur färbt. Man kann auch unter Druck bei Temperaturen über 100° C färben. Desweiteren lassen sich die Farbstoffe Spinnlösungen zur Herstellung polyacrylnitrilhaltiger Fasern zusetzen oder auch auf die unverstreckte Faser aufbringen.

Die Färbungen der erfindungsgemäßen Farbstoffe der Formel (I) auf Materialien aus Polyacrylnitril oder sauer modifizierten Polyesterfasern oder Polyamidfasern zeichnen sich durch sehr gute Licht-, Naβ-, Reib- und Sublimierechtheit und durch eine hohe Affinität zur Faser aus.

Beispiele für Verbindungen der Formel (IV) sind:

2,5-diamino-1,3,4-thiadiazol, 2-Amino-5-(di)methylamino-1,3,4-thiadiazol, 2-Amino-5-(di)-ethylamino-1,3,4-thiadiazol, 2-Amino-5-(di)n-propylamino-1,3,4-thiadiazol, 2-Amino-5-(di)-i-propylamino-1,3,4-thiadiazol, 2-Amino-5-(di)butylamino-1,3,4-thiadiazol, 2-Amino-5-(di)phenylamino-1,3,4-thiadiazol, 2-Amino-5-phenylmethylamino-1,3,4-thiadiazol, 2-Amino-5-(di)benzylamino-1,3,4-thiadiazol, 2-Amino-5-benzylmethylamino-1,3,4-thiadiazol, 2-Amino-5-phenethylmethylamino-1,3,4-thiadiazol, 2-Amino-5-morpholino-1,3,4-thiadiazol, 2-Amino-5-piperidino-1,3,4-thiadiazol, 2-Amino-5-pyrrolidino-1,3,4-thiadiazol, 2-Amino-5-(di)cyclohexylamino-1,3,4-thiadiazol, 2-Amino-5-cyclohexylmethylamino-1,3,4-thiadiazol, 2-Amino-5-hydroxyethylmethylamino-1,3,4-thiadiazol, 2-Amino-5-(di)hydroxyethylamino-1,3,4-thiadiazol, 2-Amino-5-(di)hydroxypropylmmino-1,3,4-thiadiazol, 2-Amino-5-cyanethylmethylamino-1,3,4-thiadiazol, 2-Amino-5-biscyanethylamino-1,3,4-thiadiazol.

Als Quartärsalze heterocyclischer Amine der Formel (V) können beispielsweise eingesetzt werden:

2-Amino-3-methylbenzthiazolium-, 2-Amino-3-ethylbenzthiazolium-, 2-Amino-3-methyl-6-methylbenzthiazolium-, 2-Amino-3-methyl-6-methoxybenzthiazolium-, 2-Amino-3-methyl-6-nitrobenzthiazolium-, 2-Amino-3-methyl-6-ethoxybenzthiazolium-, 2-Amino-3-methyl-1,3-thiazolium-, 2-Amino-3-methyl-5-methyl-1,3-thiazolium-, 2-Amino-3-methyl-5-ethyl-1,3-thiazolium-, 2-Amino-3-methyl-5-t-butyl-1,3-thiazolium-, 2-Amino-3-methyl-5-phenyl-1,3-thiazolium-, 2-Amino-3-methyl-4,5-dimethyl-1,3-thiazolium-, 2-Amino-3-methyl-4,5-diphenyl-1,3-thiazolium, 3-Amino-1,4-dimethyl-1,2,4-triazolium-, 3-Amino-1,4-diethyl-1,2,4-triazolium-, 3-Amino-1,4-dimethyl-5-ethyl-1,2,4-triazolium-, 3-Amino-1,4-dimethyl-5-cyanethyl-1,2,4-triazolium-, 3-Amino-1,4-dimethyl-5-butyl-1,2,4-triazolium, 3-Amino-1,4-dimethyl-5-phenyl-1,2,4-triazolium-, 3-Amino-1,4-dimethyl-5-p-tolyl-1,2,4-triazolium-, 2-Amino-5-(di)methylamino-1,3,4-thiadiazolium-, 2-Amino-5-(di)ethylamino-1,3,4-thiadiazolium-, 2-Amino-5-(di)n-propylamino-1,3,4-thiadiazolium-, 2-Amino-5-(di)-i-propylamino-1,3,4-thiadiazolium-, 2-Amino-5-(di)-n-butylamino-1,3,4-thiadiazolium-, 2-Amino-5-(di)-phenylamino-1,3,4-thiadiazolium-, 2-Amino-5-phenylmethylamino-1,3,4-thiadiazolium-, 2-Amino-5-benzylmethylamino-1,3,4-thiadiazolium-, 2-Amino-5-phenethylmethylamino-1,3,4-thiadiazolium-, 2-Amino-5-morpholino-1,3,4-thiadiazolium-, 2-Amino-5-piperidino-1,3,4-thiadiazolium-, 2-Amino-5-pyrrolidino-1,3,4-thiadiazolium-, 2-Amino-5-(di)cyclohexylamino-1,3,4-thiadiazolium-, 2-Amino-5-cyclohexylmethylamino-1,3,4-thiadiazolium-, 2-Amino-5-(di)hydroxyethylmethylamino-1,3,4-thiadiazolium-, 2-Amino-5-cyanethylmethylamino-1,3,4-thiadiazoliumsalze.

Die Quartärsalze der Formel (V) sind beispielsweise durch Umsetzung von Verbindungen der Formel (IV) mit einem Quaternierungsmittel wie z.B. Methyliodid, Ethyliodid, Propyliodid, Butyliodid, Dimethylsulfat, Ethylenoxid, Propylenoxid, Acrylnitril, Acrylamid, Acrylsäure und ihre Ester, a-Halogencarbonsäuren und ihre Ester oder Epichlorhydrin erhältlich.

Die folgenden Beispiele sollen die vorliegende Erfindung erläutern.

### Beispiel 1

In einem Autoklaven werden 20 g 2-Amino-5-diisopropylamino-1,3,4-thiadiazol, 27,6 g 2-Amino-3-methyl-1,3-benzthiazoliummethosulfat, 12 g Isoamylnitrit, 40 ml Wasser und 80 ml Methanol vereinigt, unter einen CO₂-Druck von 50 bar gesetzt und auf 40°C erwärmt. Bei dieser Temperatur wird unter konstantem Druck 3 Stunden gerührt. Dann wird der Autoklav entspannt, das Methanol abdestilliert und die Farbbase der Formel 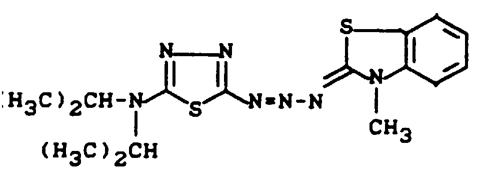 auf Wasser ausgetragen und abgesaugt. Nach dem Trocknen beträgt die Ausbeute 28,5 g, Fp: 195-198°C.

Durch Methylierung mit Dimethylsulfat erhält man einen Kationischen Farbstoff, der Polyacrylnitril in lichtechtem rotem Ton anfärbt und der Formel 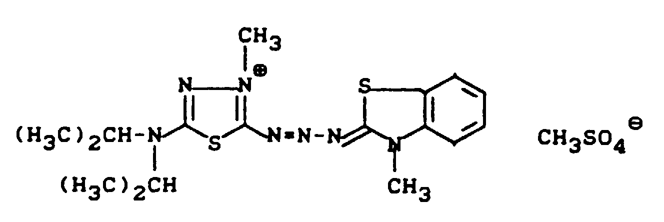 entspricht.

Ähnlich wertvolle Farbstoffe erhält man, wenn anstelle von 2-Amino-3-methyl-1,3-benzthiazoliummethosulfat die methylierten Quartärsalze von 3-Aminotriazol, von 2-Aminothiazol oder von 2-Amino-diisopropylamino-1,3,4-thidiazol für die Synthese der Farbbase einsetzt.

Analog Beispiel 1 lassen sich die in Tabelle 1 aufgeführten Farbbasen der Formel (II) herstellen, die nach Quaternierung zu den entsprechenden Farbstoffen der Formel (I) führen, die die in Tabelle 1 angegebenen Absorptionswellenlängen aufweisen.

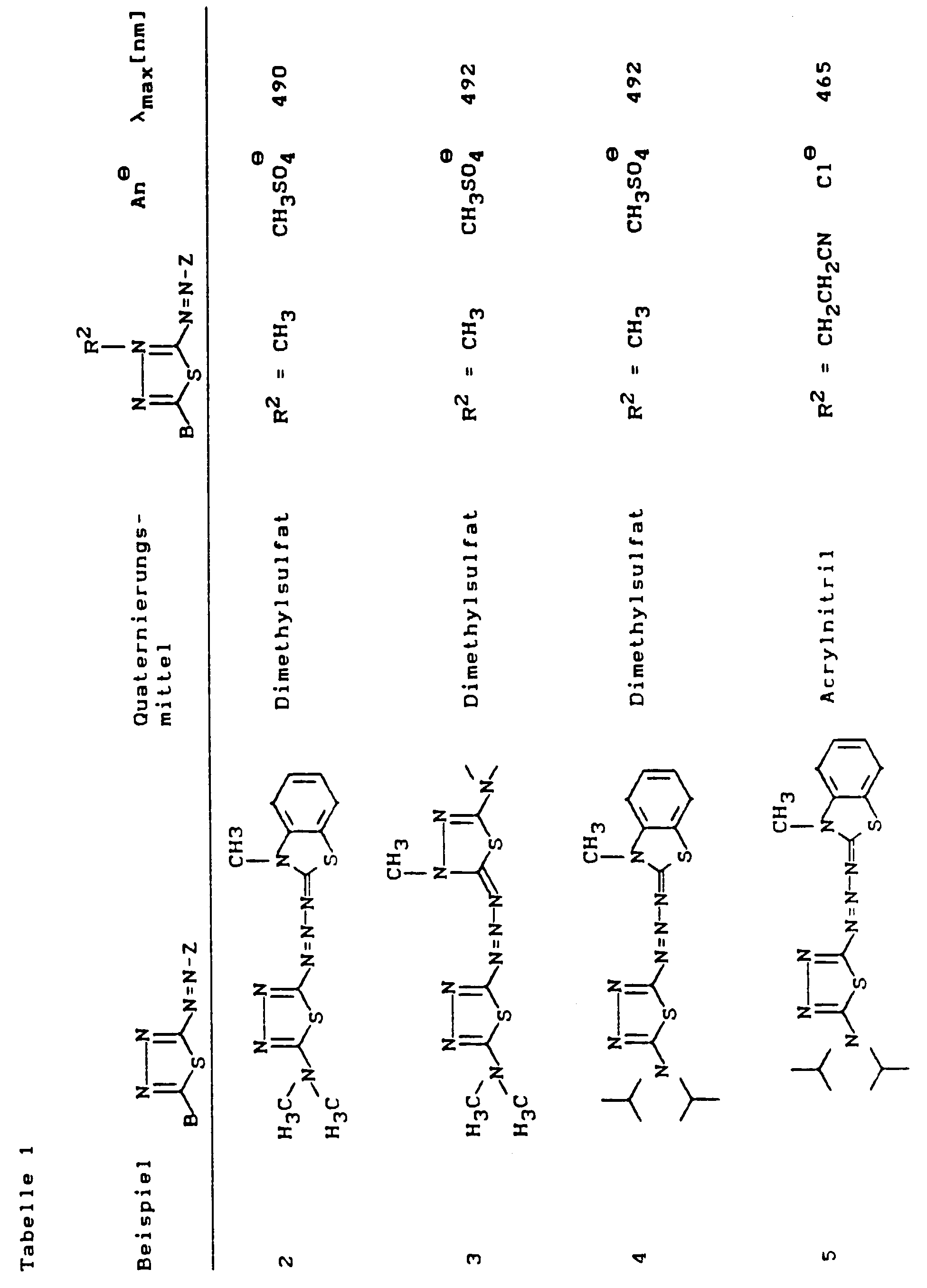

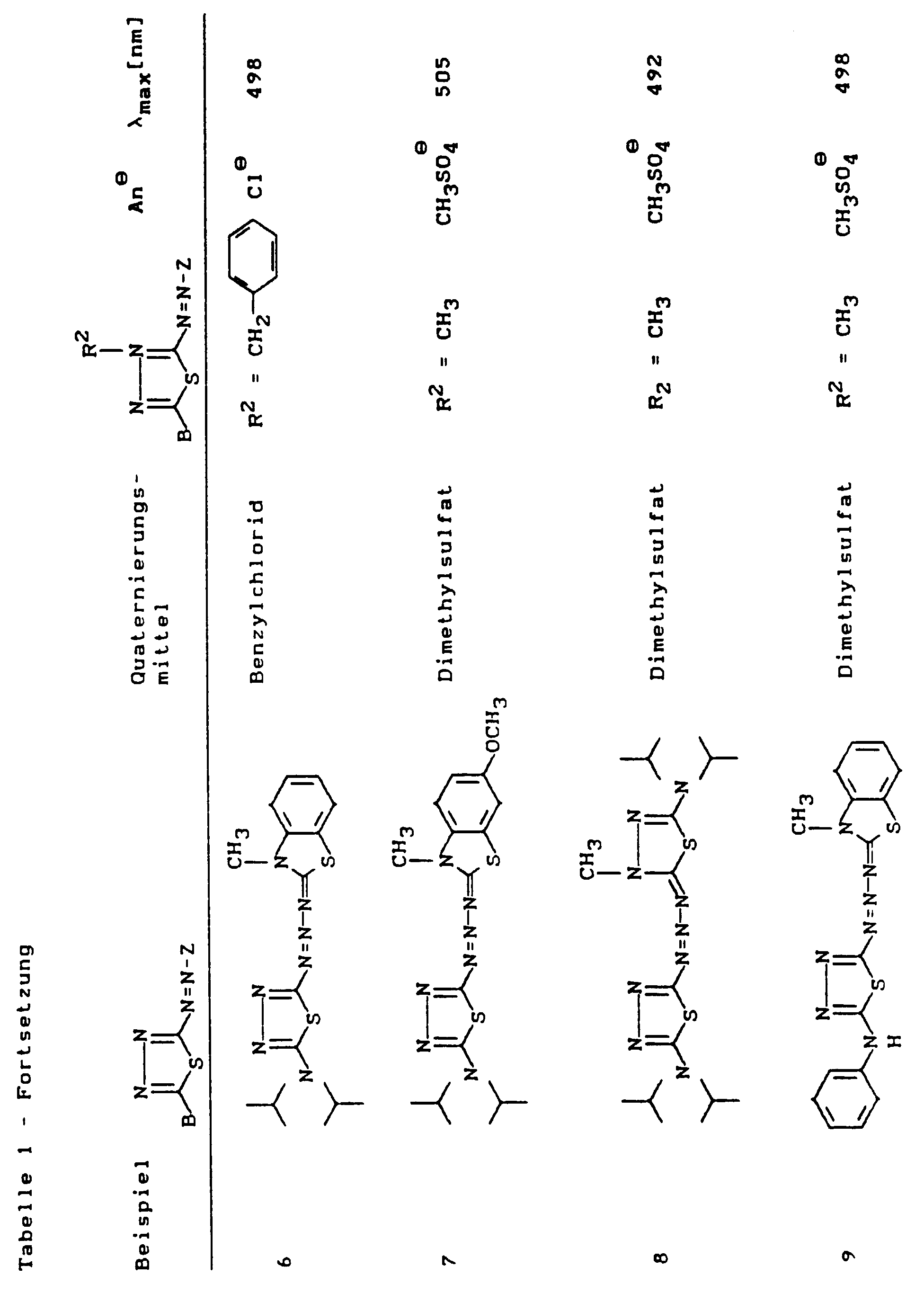

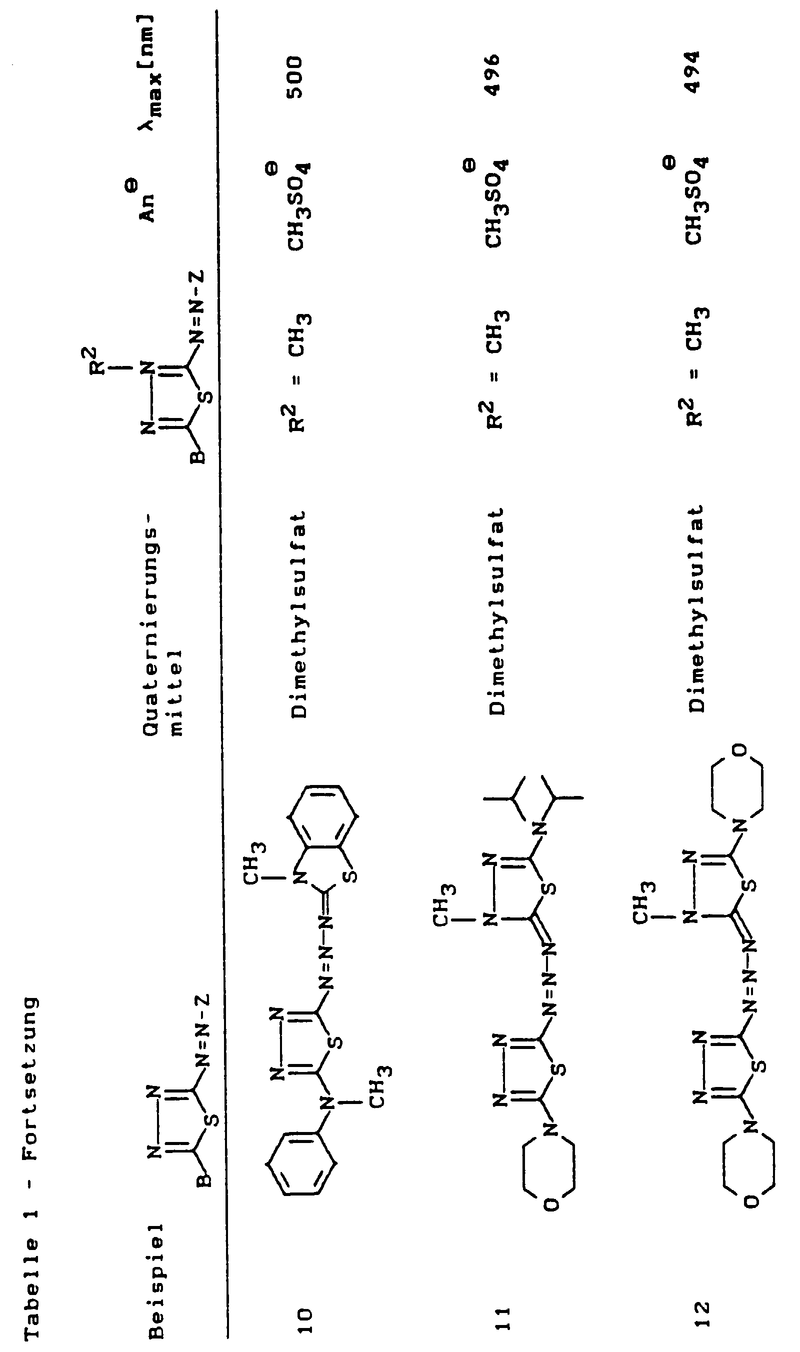

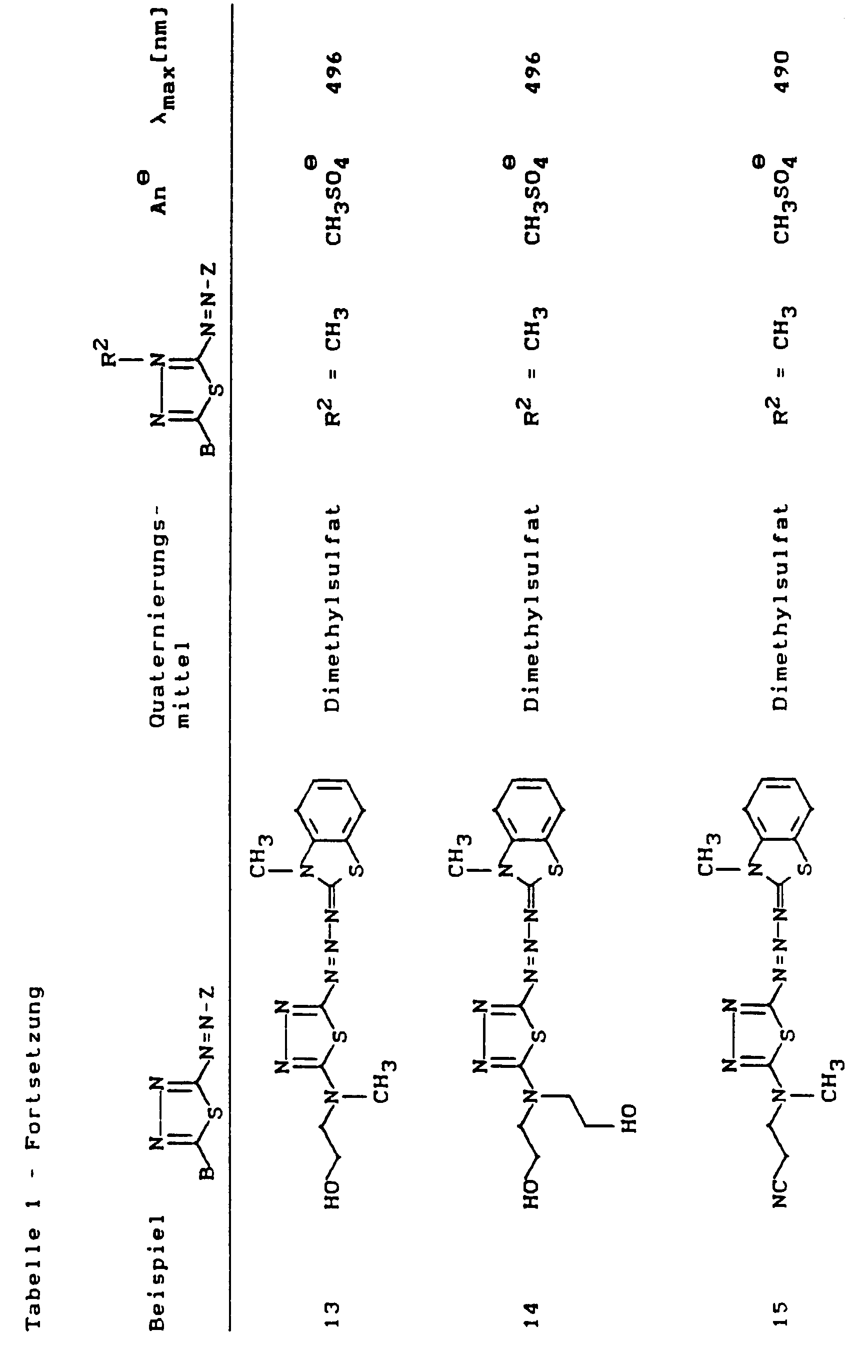

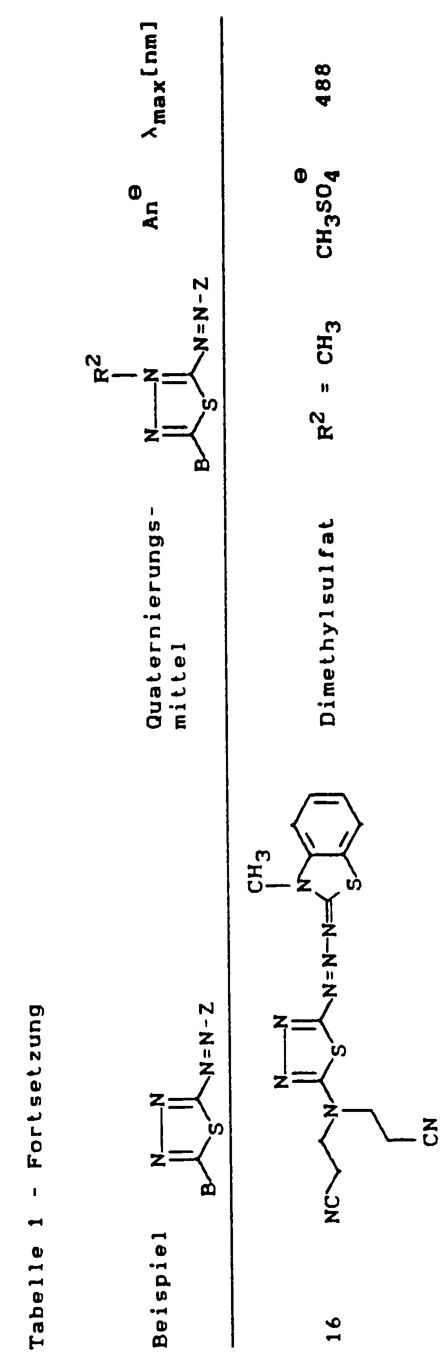

## Patentansprüche

1. Triazatrimethinfarbstoffe der Formel (I) 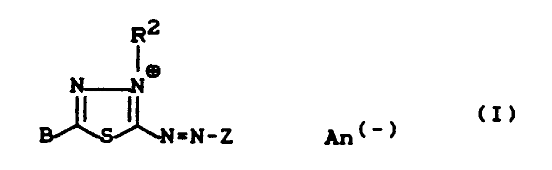 worin
Z für einen Rest der Formel 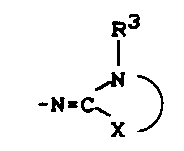
steht, worin
R³ für Wasserstoff, einen Alkyl-, Alkenyl- oder Aralkylrest steht, und
X für die restlichen Glieder eines fünfgliedrigen, gegebenenfalls substituierten und gegebenenfalls anellierten partiell ungesättigten Heterocyclus, der ein bis drei Heteroatome aus der Reihe Sauerstoff, Schwefel, Stickstoff enthält, steht
B für Alkylmercapto, Arylmercapto oder einen Rest der Formel 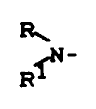
worin
R Wasserstoff, einen Alkyl-, Alkenyl-, Acyl-, Cycloalkyl-, Aryl-, Aralkyl-, Amino-, Alkylamino-, Dialkylamino-, Arylamino-, Aralkylamino- oder heterocyclischen Rest und
R¹ Wasserstoff, einen Alkyl-, Alkenyl- oder Aralkylrest bedeuten, oder
R und R¹ gemeinsam mit dem Stickstoffatom, an das sie gebunden sind, einen Heterocyclus bilden,
R₂ für Wasserstoff, einen Alkyl-, Alkenyl-, Alkinyl-oder Aralkylrest steht,
und
An⁽⁻⁾ für ein Anion steht
und worin die cyclischen und acylischen Reste nicht ionogene Substituenten und/oder eine Carboxylgruppe enthalten können.

2. Triazatrimethinfarbstoffe des Anspruchs 1, worin
Z für einen Rest der Formel 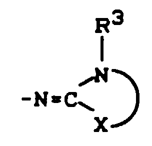
steht, worin
R³ für Wasserstoff, C₁-C₈-Alkyl, welches gegebenenfalls durch Hydroxy, Halogen, Cyano, C₁-C₄-Alkoxy, Carboxyl, C₁-C₄-(Di)-alkylamino, C₁-C₄-Alkoxycarbonyl oder C₁-C₄-Alkylsulfonyl substituiert ist, für C₂-C₄-Alkenyl oder für Phenyl-C₁-C₂-alkyl, welches gegebenenfalls durch Halogen, Cyano, C₁-C₄-Alkyl oder C₁-C₄-Alkoxy substituiert ist, und
X für die restlichen Glieder eines Thiazolin-, Isothiazolin-, Benzothiazolin-, 1,2,4-Triazolin-, 1,3,4-Thiadiazolin-, Oxazolin-, Benzoxazolin-, Imidazolin-, Benzimidazolin-, Pyrazolin- oder Benzpyrazolinrings, welcher jeweils gegebenenfalls ein- oder zweifach, gleich oder verschieden substituiert ist, durch C₁-C₈-Alkyl, welches gegebenenfalls durch Hydroxy, Halogen, Cyano, C₁-C₄-Alkoxy, Carboxyl, C₁-C₄-(Di)alkylamino, C₁-C₄-Alkoxycarbonyl oder C₁-C₄-Alkylsulfonyl substituiert ist, für C₂-C₄-Alkenyl oder für Phenyl-C₁-C₂-alkyl, welches gegebenenfalls durch Halogen, Cyano, C₁-C₄-Alkyl oder C₁-C₄-Alkoxy substituiert ist,
B für einen Rest der Formel 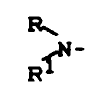
steht, worin
R für Wasserstoff, einen gegebenenfalls durch Hydroxy, Halogen, Cyan, C₁-C₄-Alkoxy, Carboxyl, Aminocarbonyl oder C₁-C₄-Alkoxycarbonyl substituierten C₁-C₈-Alkylrest, einen C₂-C₄-Alkenylrest, einen gegebenenfalls durch C₁-C₄-Alkyl substituierten Cyclohexylrest, einen gegebenenfalls durch Halogen, C₁-C₄-Alkyl oder C₁-C₄-Alkoxy substituierten Phenyl-, Benzyl-oder Phenylethylrest, einen C₁-C₄-Alkyloxycarbonyl-, Mono- oder Di-C₁-C₄-alkylaminocarbonyl-, Aminocarbonyl-, Mono- oder Di-C₁-C₄-alkylaminosulfonyl-, Amino-, C₁-C₄-Alkylamino-, Di-(C₁-C₄-alkyl)-amino-, einen gegebenenfalls durch C₁-C₄-Alkyl oder C₁-C₄-Alkoxy substituierten Phenylamino- oder Benzylamino-Rest und
R¹ für Wasserstoff, einen gegebenenfalls durch Hydroxy, Halogen, Cyan, C₁-C₄-Alkoxy, Carboxyl, Aminocarbonyl oder C₁-C₄-Alkoxycarbonyl substituierten C₁-C₈-Alkylrest, einen C₂-C₄-Alkenylrest oder einen gegebenenfalls durch Halogen, C₁-C₄-Alkyl oder C₁-C₄-Alkoxy substituierten Phenyl-, Benzyl- oder Phenylethylrest steht
und
R² für Wasserstoff, einen gegebenenfalls durch Hydroxy, Halogen, Cyan, C₁-C₄-Alkoxy, Carboxyl, Aminocarbonyl oder C₁-C₄-Alkoxycarbonyl substituierten C₁-C₈-Alkylrest, einen C₂-C₄-Alkenylrest oder einen gegebenenfalls durch Halogen, C₁-C₄-Alkyl oder C₁-C₄-Alkoxy substituierten Benzyl-oder Phenylethylrest steht.

3. Farbstoffe des Anspruchs 1 worin
Z für einen Rest der Formel 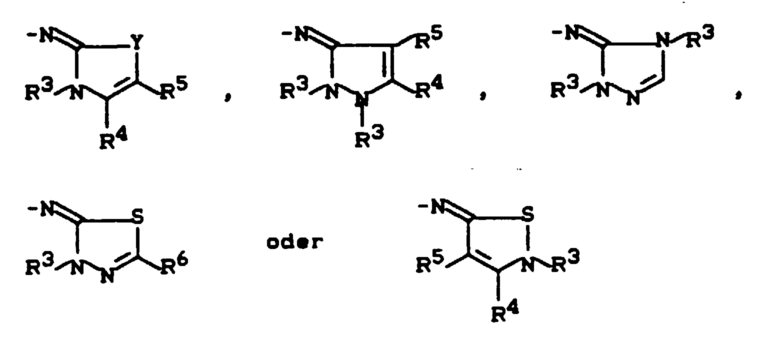
steht worin
Y für -S-, -O- oder 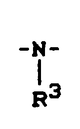 steht,
wobei R³ die oben angegebene Bedeutung hat, und beim Auftreten von zwei Substituenten R³ diese gleich oder verschieden sein können,
R⁴ und R⁵ entweder für Wasserstoff stehen, oder gemeinsam für die restlichen Glieder eines anellierten Benzolrings stehen, und
R⁶ für C₁-C₄-(Di)alkylamino, C₆-C₁₀-(Di)arylamino, Piperazino, Pyrrolidino, C₁-C₄-Alkylmercapto oder C₆-C₁₀-Arylmercapto steht,
B für einen Rest der Formel 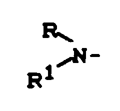 steht,
worin
R für Wasserstoff, einen gegebenenfalls durch Hydroxy, Halogen, Cyan, C₁-C₄-Alkoxy, Carbonyl, Aminocarbonyl oder C₁-C₄-Alkoxycarbonyl substituierten C₁-C₄-Alkylrest, einen gegebenenfalls durch Halogen, C₁-C₄-Alkyl oder C₁-C₄-Alkoxy substituierten Phenyl-, Benzyl-oder Phenylethylrest,
R¹ für einen gegebenenfalls durch Hydroxy, Halogen, Cyan, C₁-C₄-Alkoxy, Carboxy, Aminocarbonyl oder C₁-C₄-Alkoxycarbonyl substituierten C₁-C₄-Alkylrest oder einen gegebenenfalls durch Halogen, C₁-C₄-Alkyl oder C₁-C₄-Alkoxy substituierten Benzyl- oder Phenylethylrest steht
und
R² für Wasserstoff, einen gegebenenfalls durch Hydroxy, Halogen, Cyan, C₁-C₄-Alkoxy, Carboxy, Aminocarbonyl oder C₁-C₄-Alkoxycarbonyl substituierten C₁-C₈-Alkylrest oder einen gegebenenfalls durch Halogen, C₁-C₄-Alkyl oder C₁-C₄-Alkoxy substituierten Benzyl- oder Phenylethylrest steht.

4. Farbstoffe der Formel 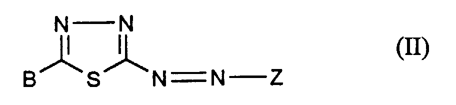 worin
Z für einen Rest der Formel 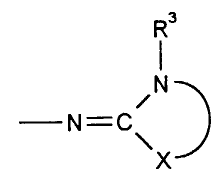
steht, worin
R³ für Wasserstoff, einen Alkyl-, Alkenyl- oder Aralkylrest steht, und
X für die restlichen Glieder eines fünfgliedrigen, gegebenenfalls substituierten und gegebenenfalls anellierten partiell ungesättigten Heterocyclus, der ein bis drei Heteroatome aus der Reihe Sauerstoff, Schwefel, Stickstoff enthält, steht und
B für Alkylmercapto, Arylmercapto oder einen Rest der Formel 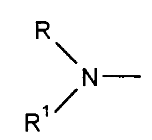
steht, worin
R Wasserstoff, einen Alkyl-, Alkenyl-, Acyl-, Cycloalkyl-, Aryl-, Aralkyl-, Amino-, Alkylamino-, Dialkylamino-, Arylamino-, Aralkylamino- oder heterocyclischen Rest und
R¹ Wasserstoff, einen Alkyl-, Alkenyl- oder Aralkylrest bedeuten, oder
R und R¹ gemeinsam mit dem Stickstoffatom, an das sie gebunden sind, einen Heterocyclus bilden.

5. Farbstoffe des Anspruchs 4, worin
Z für einen Rest der Formel 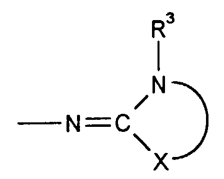
steht, worin
R³ für Wasserstoff, C₁-C₈-Alkyl, welches gegebenenfalls durch Hydroxy, Halogen, Cyano, C₁-C₄-Alkoxy, Carboxyl, C₁-C₄-(Di)-alkylamino, C₁-C₄-Alkoxycarbonyl oder C₁-C₄-Alkylsulfonyl substituiert ist, für C₂-C₄-Alkenyl oder für Phenyl-C₁-C₂-alky-, welches gegebenenfalls durch Halogen, Cyano, C₁-C₄-Alkyl oder C₁-C₄-Alkoxy substituiert ist, und
X für die restlichen Glieder eines Thiazolin-, Isothiazolin-, Benzothiazolin-, 1,2,4-Triazolin-, 1,3,4-Thiadiazolin-, Oxazolin-, Benzoxazolin-, Imidazolin-, Benzimidazolin-, Pyrazolin- oder Benzpyrazolinrings, welcher jeweils gegebenenfalls ein- oder zweifach, gleich oder verschieden substituiert ist, durch C₁-C₈-Alkyl, welches gegebenenfalls durch Hydroxy, Halogen, Cyano, C₁-C₄-Alkoxy, Carboxyl, C₁-C₄-(Di)-alkylamino, C₁-C₄-Alkoxycarbonyl oder C₁-C₄-Alkylsulfonyl substituiert ist, für C₂-C₄-Alkenyl oder für Phenyl-C₁-C₂-alkyl, welches gegebenenfalls durch Halogen, Cyano, C₁-C₄-Alkyl oder C₁-C₄-Alkoxy substituiert ist, und
B für einen Rest der Formel 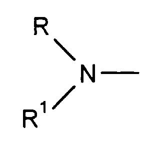 steht, worin
R für Wasserstoff, einen gegebenenfalls durch Hydroxy, Halogen, Cyan, C₁-C₄-Alkoxy, Carboxyl, Aminocarbonyl oder C₁-C₄-Alkoxycarbonyl substituierten C₁-C₈-Alkylrest, einen C₂-C₄-Alkenylrest, einen gegebenenfalls durch C₁-C₄-Alkyl substituierten Cyclohexylrest, einen gegebenenfalls durch Halogen, C₁-C₄-Alkyl oder C₁-C₄-Alkoxy substituierten Phenyl-, Benzyl- oder Phenylethylrest, einen C₁-C₄-Alkyloxycarbonyl-, Mono oder Di-C₁-C₄-alkylaminocarbonyl-, Aminocarbonyl-, Mono- oder Di-C₁-C₄-alkylaminosulfonyl-, Amino-, C₁-C₄-Alkylamino-, Di-(C₁-C₄-alkyl)-amino-, einen gegebenenfalls durch C₁-C₄-Alkyl oder C₁-C₄-Alkoxy substituierten Phenylamino- oder Benzylamino-Rest und
R¹ für Wasserstoff, einen gegebenenfalls durch Hydroxy, Halogen, Cyan, C₁-C₄-Alkoxy, Carboxyl, Aminocarbonyl oder C₁-C₄-Alkoxycarbonyl substituierten C₁-C₈-Alkylrest, einen C₂-C₄-Alkenylrest, einen gegebenenfalls durch Halogen, C₁-C₄-Alkyl oder C₁-C₄-Alkoxy substituierten Phenyl-, Benzyl- oder Phenylethylrest steht.

6. Verfahren zur Herstellung der Farbstoffe des Anspruchs 4, dadurch gekennzeichnet, daß eine Verbindung der Formel (IV) 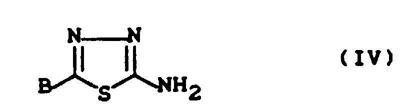 worin
B die in Anspruch 4 angegebene Bedeutung hat,
und eine Verbindung der Formel (V)
H-Z¹ (V)
worin
Z¹ für einen Rest der Formel 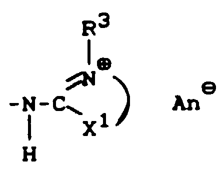
steht, worin
R³ für Wasserstoff, einen Alkyl-, Alkenyl- oder Aralkylrest steht,
An^{⊖} für ein Anion steht, und
X¹ für die restlichen Glieder eines quaternierten fünfgliedrigen gegebenenfalls substituierten und gegebenenfalls anellierten, ungesättigten Heterocyclus, der ein bis drei Heteroatome aus der Reihe Sauerstoff, Schwefel, Stickstoff enthält, steht,
in wäßrigem Medium mit einer salpetrige Säure abgebenden Substanz in Gegenwart von CO₂ unter einem Druck von 5 bis 100 bar bei einer Temperatur von 0 bis 125°C umgesetzt werden.

7. Verfahren gemäß Anspruch 6, dadurch gekennzeichnet, daß es sich bei der salpetrigen Säure abgebenden Substanz um ein anorganisches oder organisches Nitrit handelt.

8. Verfahren zum Färben und Bedrucken von natürlichen und synthetischen Materialien, insbesondere von Fasern aus Polyacrylnitril, sauer modifizierten Polyestern und Polyamiden, dadurch gekennzeichnet, daß Farbstoffe des Anspruchs 1 eingesetzt werden.

9. Verfahren zum Färben und Bedrucken von natürlichen und synthetischen Materialien, insbesondere von Fasern und Geweben aus mit nichtionischen Farbstoffen anfärbbaren Materialien, dadurch gekennzeichnet, daß Farbstoffe des Anspruchs 4 eingesetzt werden.

10. Verfahren zum Massefärben von Kunststoffen, dadurch gekennzeichnet, daß Farbstoffe des Anspruchs 4 eingesetzt werden.

11. Verfahren zur Herstellung von Triazatrimethinfarbstoffen gemäß Anspruch 1, dadurch gekennzeichnet, daß man Farbbasen der Formel (II) 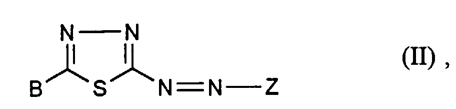 worin
B und Z die in Anspruch 1 angegebenen Bedeutungen haben, mit einem Alkylierungsmittel aus der Klasse der Alkylhalogenide, Michael-Akzeptoren oder Epoxide, umsetzt.

## Claims

1. Triazatrimethine dyestuffs of the formula (I) 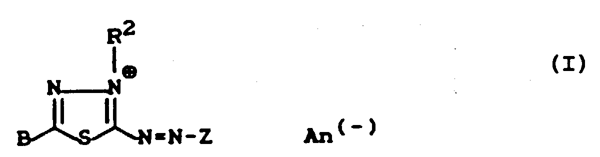 wherein
Z represents a radical of the formula 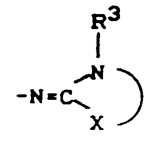
wherein
R³ represents hydrogen or an alkyl, alkenyl or aralkyl radical and
X represents the remaining members of a five-membered, optionally substituted and optionally fused partly unsaturated heterocyclic radical which contains one to three hetero atoms from the series comprising oxygen, sulphur and nitrogen,
B represents alkylmercapto, arylmercapto or a radical of the formula 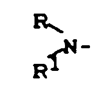
wherein
R denotes hydrogen or an alkyl, alkenyl, acyl, cycloalkyl, aryl, aralkyl, amino, alkylamino, dialkylamino, arylamino, aralkylamino or heterocyclic radical and
R¹ denotes hydrogen or an alkyl, alkenyl or aralkyl radical, or
R and R¹, together with the nitrogen atom to which they are bonded, form a heterocyclic radical,
R₂ represents hydrogen or an alkyl, alkenyl, alkinyl or aralkyl radical
and
An⁽⁻⁾ represents an anion,
and wherein the cyclic and acyclic radicals can contain non-ionic substituents and/or a carboxyl group.

2. Triazatrimethine dyestuffs of Claim 1, wherein
Z represents a radical of the formula 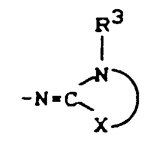 wherein
R³ represents hydrogen, C₁-C₈-alkyl, which is optionally substituted by hydroxyl, halogen, cyano, C₁-C₄-alkoxy, carboxyl, C₁-C₄-(di)-alkylamino, C₁-C₄-alkoxycarbonyl or C₁-C₄-alkylsulphonyl, or represents C₂-C₄-alkenyl, or represents phenyl-C₁-C₂-alkyl, which is optionally substituted by halogen, cyano, C₁-C₄-alkyl or C₁-C₄-alkoxy and
X represents the remaining members of a thiazoline, isothiazoline, benzothiazoline, 1,2,4-triazoline, 1,3,4-thiadiazoline, oxazoline, benzoxazoline, imidazoline, benzimidazoline, pyrazoline or benzopyrazoline ring, which is in each case optionally mono- or disubstituted by identical or different C₁-C₈-alkyl, which is optionally substituted by hydroxyl, halogen, cyano, C₁-C₄-alkoxy, carboxyl, C₁-C₄-(di)alkylamino, C₁-C₄-alkoxycarbonyl or C₁-C₄-alkylsulphonyl, or represents C₂-C₄-alkenyl, or represents phenyl-C₁-C₂-alkyl, which is optionally substituted by halogen, cyano, C₁-C₄-alkyl or C₁-C₄-alkoxy,
B represents a radical of the formula 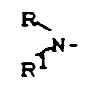
wherein
R represents hydrogen, a C₁-C₈-alkyl radical which is optionally substituted by hydroxyl, halogen, cyano, C₁-C₄-alkoxy, carboxyl, aminocarbonyl or C₁-C₄-alkoxycarbonyl, a C₂-C₄-alkenyl radical, a cyclohexyl radical which is optionally substituted by C₁-C₄-alkyl, a phenyl, benzyl or phenylethyl radical which is optionally substituted by halogen, C₁-C₄-alkyl or C₁-C₄-alkoxy, a C₁-C₄-alkyloxycarbonyl, mono- or di-C₁-C₄-alkylaminocarbonyl, aminocarbonyl, mono- or di-C₁-C₄-alkylaminosulphonyl, amino, C₁-C₄-alkylamino or di-(C₁-C₄-alkyl)-amino radical, or a phenylamino or benzylamino radical which is optionally substituted by C₁-C₄-alkyl or C₁-C₄-alkoxy and
R¹ represents hydrogen, a C₁-C₈-alkyl radical which is optionally substituted by hydroxyl, halogen, cyano, C₁-C₄-alkoxy, carboxyl, aminocarbonyl or C₁-C₄-alkoxycarbonyl, a C₂-C₄-alkenyl radical or a phenyl, benzyl or phenylethyl radical which is optionally substituted by halogen, C₁-C₄-alkyl or C₁-C₄-alkoxy,
and
R² represents hydrogen, a C₁-C₈-alkyl radical which is optionally substituted by hydroxyl, halogen, cyano, C₁-C₄-alkoxy, carboxyl, aminocarbonyl or C₁-C₄-alkoxycarbonyl, a C₂-C₄-alkenyl radical or a benzyl or phenylethyl radical which is optionally substituted by halogen, C₁-C₄-alkyl or C₁-C₄-alkoxy.

3. Dyestuffs of Claim 1, wherein
Z represents a radical of the formula 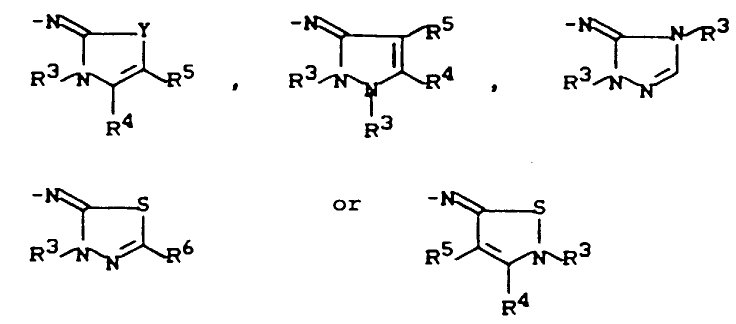
wherein
y represents -S-, -O- or 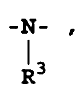 wherein R³ has the abovementioned meaning, and if two substituents R³ occur, these can be identical or different,
R⁴ and R⁵ either represent hydrogen, or together represent the remaining members of a fused-on benzene ring, and
R⁶ represents C₁-C₄-(di)alkylamino, C₆-C₁₀-(di)arylamino, piperazino, pyrrolidino, C₁-C₄-alkylmercapto or C₆-C₁₀-arylmercapto,
B represents a radical of the formula 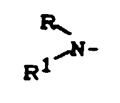 wherein
R represents hydrogen, a C₁-C₄-alkyl radical which is optionally substituted by hydroxyl, halogen, cyano, C₁-C₄-alkoxy, carbonyl, aminocarbonyl or C₁-C₄-alkoxycarbonyl, or a phenyl, benzyl or phenylethyl radical which is optionally substituted by halogen, C₁-C₄-alkyl or C₁-C₄-alkoxy and
R¹ represents a C₁-C₄-alkyl radical which is optionally substituted by hydroxyl, halogen, cyano, C₁-C₄-alkoxy, carboxyl, aminocarbonyl or C₁-C₄-alkoxycarbonyl, or a benzyl or phenylethyl radical which is optionally substituted by halogen, C₁-C₄-alkyl or C₁-C₄-alkoxy,
and
R² represents hydrogen, a C₁-C₈-alkyl radical which is optionally substituted by hydroxyl, halogen, cyano, C₁-C₄-alkoxy, carboxyl, aminocarbonyl or C₁-C₄-alkoxycarbonyl or a benzyl or phenylethyl radical which is optionally substituted by halogen, C₁-C₄-alkyl or C₁-C₄-alkoxy.

4. Dyestuffs of the formula 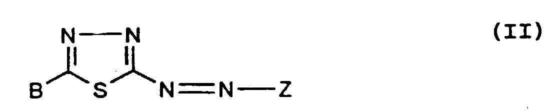 wherein
Z represents a radical of the formula 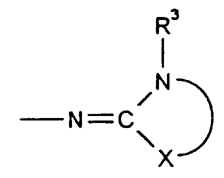
wherein
R³ represents hydrogen or an alkyl, alkenyl or aralkyl radical and
X represents the remaining members of a five-membered, optionally substituted and optionally fused partly unsaturated heterocyclic radical which contains one to three hetero atoms from the series comprising oxygen, sulphur and nitrogen,
B represents alkylmercapto, arylmercapto or a radical of the formula 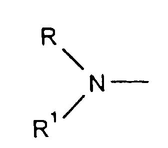
wherein
R denotes hydrogen or an alkyl, alkenyl, acyl, cycloalkyl, aryl, aralkyl, amino, alkylamino, dialkylamino, arylamino, aralkylamino or heterocyclic radical and
R¹ denotes hydrogen or an alkyl, alkenyl or aralkyl radical, or
R and R¹, together with the nitrogen atom to which they are bonded, form a heterocyclic radical.

5. Dyestuffs of Claim 4, wherein
Z represents a radical of the formula 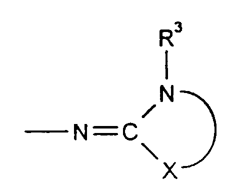
wherein
R³ represents hydrogen, C₁-C₈-alkyl, which is optionally substituted by hydroxyl, halogen, cyano, C₁-C₄-alkoxy, carboxyl, C₁-C₄-(di)-alkylamino, C₁-C₄-alkoxycarbonyl or C₁-C₄-alkylsulphonyl, or represents C₂-C₄-alkenyl, or represents phenyl-C₁-C₂-alkyl, which is optionally substituted by halogen, cyano, C₁-C₄-alkyl or C₁-C₄-alkoxy and
X represents the remaining members of a thiazoline, isothiazoline, benzothiazoline, 1,2,4-triazoline, 1,3,4-thiadiazoline, oxazoline, benzoxazoline, imidazoline, benzimidazoline, pyrazoline or benzopyrazoline ring, which is in each case optionally mono- or disubstituted by identical or different C₁-C₈-alkyl, which is optionally substituted by hydroxyl, halogen, cyano, C₁-C₄-alkoxy, carboxyl, C₁-C₄-(di)-alkylamino, C₁-C₄-alkoxycarbonyl or C₁-C₄-alkylsulphonyl, or represents C₂-C₄-alkenyl, or represents phenyl-C₁-C₂-alkyl, which is optionally substituted by halogen, cyano, C₁-C₄-alkyl or C₁-C₄-alkoxy,
B represents a radical of the formula 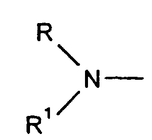
wherein
R represents hydrogen, a C₁-C₈-alkyl radical which is optionally substituted by hydroxyl, halogen, cyano, C₁-C₄-alkoxy, carboxyl, aminocarbonyl or C₁-C₄-alkoxycarbonyl, a C₂-C₄-alkenyl radical, a cyclohexyl radical which is optionally substituted by C₁-C₄-alkyl, a phenyl, benzyl or phenylethyl radical which is optionally substituted by halogen, C₁-C₄-alkyl or C₁-C₄-alkoxy, a C₁-C₄-alkyloxycarbonyl, mono- or di-C₁-C₄-alkylaminocarbonyl, aminocarbonyl, mono- or di-C₁-C₄-alkylaminosulphonyl, amino, C₁-C₄-alkylamino or di-(C₁-C₄-alkyl)-amino radical, or a phenylamino or benzylamino radical which is optionally substituted by C₁-C₄-alkyl or C₁-C₄-alkoxy and
R¹ represents hydrogen, a C₁-C₈-alkyl radical which is optionally substituted by hydroxyl, halogen, cyano, C₁-C₄-alkoxy, carboxyl, aminocarbonyl or C₁-C₄-alkoxycarbonyl, a C₂-C₄-alkenyl radical or a phenyl, benzyl or phenylethyl radical which is optionally substituted by halogen, C₁-C₄-alkyl or C₁-C₄-alkoxy.

6. Process for the preparation of the dyestuffs of Claim 4, characterised in that a compound of the formula (IV) 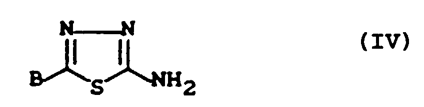 wherein
B has the meaning given in Claim 4,
and a compound of the formula (V)
H-Z¹ (V)
wherein
Z¹ represents a radical of the formula 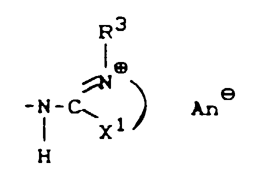
wherein
R³ represents hydrogen or an alkyl, alkenyl or aralkyl radical,
An^{⊖} represents an anion and
X¹ represents the remaining members of a quaternised five-membered optionally substituted and optionally fused, unsaturated heterocyclic radical which contains 1 to 3 hetero atoms from the series comprising oxygen, sulphur and nitrogen,
are reacted in an aqueous medium with a substance which donates nitrous acid, in the presence of CO₂ under a pressure of 5 to 100 bar and at a temperature of 0 to 125°C.

7. Process according to Claim 6, characterised in that the substance which donates nitrous acid is an inorganic or organic nitrate.

8. Process for dyeing and printing naturally occurring and synthetic materials, in particular fibres of polyacrylonitrile, acid-modified polyesters and polyamides, characterised in that the dyestuffs of Claim 1 are employed.

9. Process for dyeing and printing naturally occurring and synthetic materials, in particular fibres and fabrics of materials which can be dyed with non-ionic dyestuffs, characterised in that dyestuffs of Claim 4 are employed.

10. Process for bulk dyeing of plastics, characterised in that dyestuffs of Claim 4 are employed.

11. Process for the preparation of triazatrimethine dyes according to Claim 1, characterised in that colour bases of the formula (II) 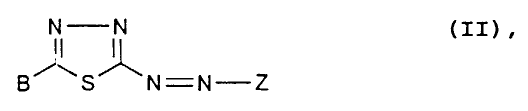 wherein
B and Z have the meanings given in Claim 1, are reacted with an alkylating agent from the class comprising alkyl halides, Michael acceptors or epoxides.

## Revendications

1. Colorants de triazatriméthine de formule (I) 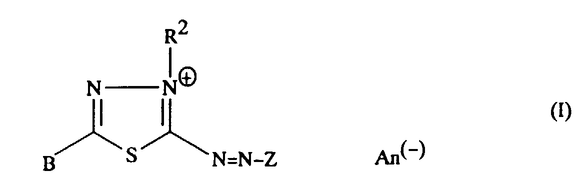 dans laquelle
Z représente un groupe de formule 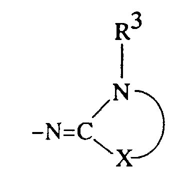
dans laquelle
R³ représente l'hydrogène, un groupe alkyle, alcényle ou aralkyle et
X représente les chaînons manquants d'un hétérocycle partiellement insaturé à 5 chaînons, éventuellement substitué et éventuellement condensé, qui contient 1 à 3 hétéroatomes choisis parmi l'oxygène, le soufre et l'azote,
B représente un groupe alkylmercapto, arylmercapto ou le groupe de formule 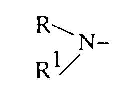
dans laquelle
R représente l'hydrogène, un groupe alkyle, alcényle, acyle, cycloalkyle, aryle, aralkyle, amino, alkylamino, dialkylamino, arylamino, aralkylamino ou un groupe hétérocyclique, et
R¹ représente l'hydrogène, un groupe alkyle, alcényle ou aralkyle, ou bien
R et R¹ forment ensemble et avec l'atome d'azote auquel ils sont reliés, un hétérocycle,
R² représente l'hydrogène, un groupe alkyle, alcényle alcynyle ou aralkyle et
An⁽⁻⁾ représente un anion,
et dans laquelle les groupes cycliques et acycliques peuvent contenir des substituants non ionogènes et/ou un groupe carboxyle.

2. Colorants de triazatriméthine selon la revendication 1,
pour lesquels
Z représente un groupe de formule 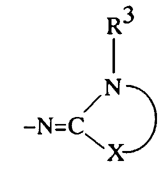
dans laquelle
R³ représente l'hydrogène, un groupe alkyle en C₁ à C₈ éventuellement substitué par des groupes hydroxy, des halogènes, des groupes cyano, alcoxy en C₁ à C₄, carboxyle, di(alkyle en C₁ à C₄)amino, (alcoxy en C₁ à C₄)carbonyle ou alkylsulfonyle en C₁ à C₄, un groupe alcényle en C₂ à C₄ ou un groupe phényl-alkyle en C₁ à C₂ qui peut être substitué par des halogènes, des groupcs cyano, alkyle en C₁ à C₄ ou alcoxy en C₁ à C₄ et
X représente les chaînons manquants d'un cycle thiazoline, isothiazoline, benzothiazoline, 1,2,4-triazoline, 1,3,4-thiadiazoline, oxazoline, benzoxazoline, imidazoline, benzimidazoline, pyrazoline ou benzopyrazoline qui peuvent chacun porter le cas échéant un ou deux substituants identiques ou différents choisis parmi les groupes alkyle en C₁ à C₈ eux-mêmes éventuellement substitués par des groupes hydroxy, des halogènes, des groupes cyano, alcoxy en C₁ à C₄, carboxyle, di(alkyle en C₁ à C₄)amino, (alcoxy en C₁ à C₄)carbonyle ou alkylsulfonyle en C₁ à C₄, les groupes alcényle en C₂ à C₄ ou les groupes phényl-alkyle en C₁ à C₂ qui peuvent ,le cas échéant, être substitués par des halogènes, des groupes cyano, alkyle en C₁ à C₄ ou alcoxy en C₁ à C₄,
B représente le groupe de formule 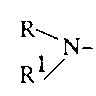
dans laquelle
R représente l'hydrogène, un groupe alkyle en C₁ à C₈ éventuellement substitué par des groupes hydroxy, des halogènes, des groupes cyano, alcoxy en C₁ à C₄, carboxyle, aminocarbonyle ou (alcoxy en C₁ à C₄)carbonyle, un groupe alcényle en C₂-C₄, un groupe cyclohexyle éventuellement substitué par des groupes alkyle en C₁ à C₄, un groupe phényle, benzyléthyle éventuellement substitué par des halogènes, des groupes alkyle en C₁ à C₄ ou alcoxy en C₁ à C₄, un groupe (alkyle en C₁ à C₄)oxycarbonyle, mono- ou di-(alkyle en C₁ à C₄)aminocarbonyle, aminocarbonyle, mono- ou di-(alkyle en C₁ à C₄)aminosulfonyle, amino, alkylamino en C₁ à C₄, di(alkyle en C₁ à C₄)amino, un groupe phénylamino ou benzylamino éventuellement substitué par des groupes alkyle en C₁ à C₄ ou alcoxy en C₁ à C₄ et
R¹ représente l'hydrogène, un groupe alkyle en C₁ à C₈ éventuellement substitué par des groupes hydroxy, des halogènes, des groupes cyano, alcoxy en C₁ à C₄, carboxyle, aminocarbonyle ou (alcoxy en C₁ à C₄)carbonylc, un groupe alcényle en C₂-C₄ ou un groupe phényle, benzyle ou phényléthyle éventuellement substitué par des halogènes, des groupes alkyle en C₁ à C₄ ou alcoxy en C₁ à C₄,
et
R² représente l'hydrogène, un groupe alkyle en C₁ à C₈ éventuellement substitué par des groupes hydroxy, des halogènes, des groupes cyano, alcoxy en C₁ à C₄, carboxyle, aminocarbonyle ou (alcoxy en C₁ à C₄)carbonyle, un groupe alcényle en C₂-C₄ ou un groupe benzyle ou phényléthyle éventuellement substitué par des halogènes, des groupe alkyles en C₁ à C₄ ou alcoxy en C₁ à C₄.

3. Colorants selon la revendication 1, pour lesquels
Z représente un groupe de formule 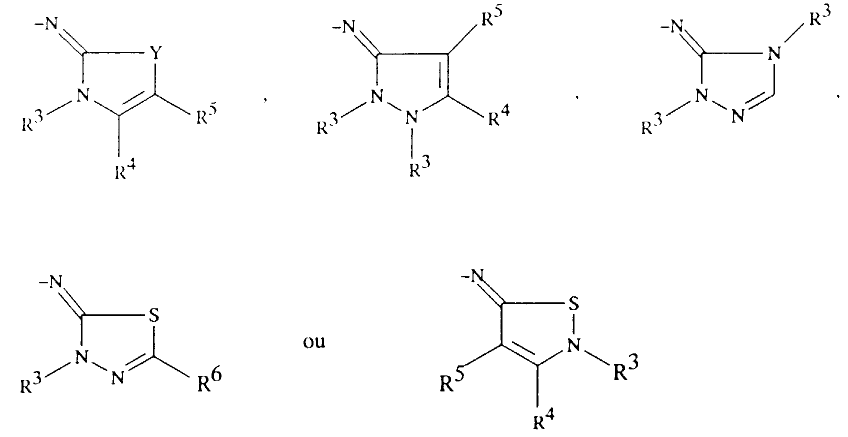
dans lesquelles
Y représente -S-, -O- ou 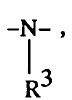 R³ ayant les significations indiquées ci-dessus, sous réserve que, lorsqu'il y a deux substituants R³, ceux-ci peuvent être identiques ou différents,
R⁴ et R⁵ représentent l'hydrogène ou bien, ensemble, les chaînons manquants d'un cycle benzénique condensé, et
R⁶ représente un groupe di(alkyle en C₁ à C₄)amino, di(aryle en C₆-C₁₀)amino, pipérazino, pyrrolidino, alkylmercapto en C₁ à C₄ ou arylmercapto en C₆-C₁₀,
B représente le groupe de formule 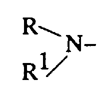 dans laquelle
R représente l'hydrogène, un groupe alkyle en C₁ à C₄ éventuellement substitué par des groupes hydroxy, des halogènes, des groupes cyano, alcoxy en C₁ à C₄, carbonyle, aminocarbonyle ou (alcoxy en C₁ à C₄)carbonyle, un groupe phényle, benzyle ou phényléthyle éventuellement substitué par des halogènes, des groupes alkyle en C₁ à C₄ ou alcoxy en C₁ à C₄,
R¹ représente un groupe alkyle en C₁ à C₄ éventuellement substitué par des groupes hydroxy, des halogènes, dcs groupes cyano, alcoxy en C₁ à C₄, carboxy, aminocarbonyle ou (alcoxy en C₁ à C₄)carbonyle, ou bien un groupe benzyle ou phényléthyle éventuellement substitué par des halogènes, des groupes alkyle en C₁ à C₄ ou alcoxy en C₁ à C₄,
et
R² représente l'hydrogène, un groupe alkyle en C₁ à C₈ éventuellement substitué par des groupes hydroxy, des halogènes, des groupes cyano, alcoxy en C₁ à C₄, carboxy, aminocarbonyle ou (alcoxy en C₁ à C₄)carbonyle ou bien un groupe benzyle ou phényléthyle éventuellement substitué par des halogènes, des groupes alkyle en C₁ à C₄ ou alcoxy en C₁ à C₄.

4. Colorants de formule 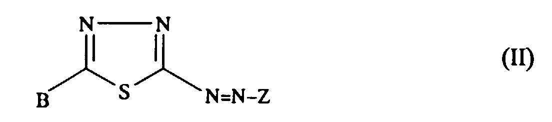 dans laquelle
Z représente un groupe de formule 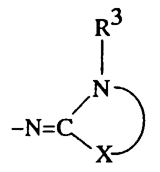
dans laquelle
R³ représente l'hydrogène, un groupe alkyle, alcényle ou aralkyle et
X représente les chaînons manquants d'un hétérocycle à 5 chaînons, partiellement insaturé, éventuellement substitué et éventuellement condensé, qui contient 1 à 3 hétéroatomes choisis parmi l'oxygène, le soufre et l'azote, et
B représente un groupe alkylmercapto, arylmercapto ou le groupe de formule 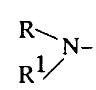
dans laquelle
R représente l'hydrogène, un groupe alkyle, alcényle, acyle, cycloalkyle, aryle, aralkyle, amino, alkylamino, dialkylamino, arylamino, aralkylamino ou un groupe hétérocyclique, et
R¹ représente l'hydrogène, un groupe alkyle, alcényle ou aralkyle, ou bien
R et R¹ forment ensemble et avec l'atome d'azote auquel ils sont reliés, un hétérocycle.

5. Colorants de la revendication 4, pour lesquels
Z représente un groupe de formule 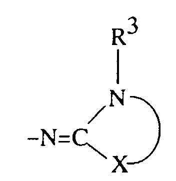 dans laquelle
R³ représente l'hydrogène, un groupe alkyle en C₁ à C₈ éventuellement substitué par des groupes hydroxy, des halogènes, des groupes cyano, alcoxy en C₁ à C₄, carboxyle, di(alkyle en C₁ à C₄)amino, (alcoxy en C₁ à C₄)carbonyle ou alkylsulfonyle en C₁ à C₄, un groupe alcényle en C₂ à C₄ ou un groupe phényl-alkyle en C₁ à C₂ éventuellement substitué par des halogènes, des groupes cyano, alkyle en C₁ à C₄ ou alcoxy en C₁ à C₄ et
X représente les chaînons manquants d'un cycle thiazoline, isothiazoline, benzothiazoline, 1,2,4-triazoline, 1,3,4-thiadiazoline, oxazoline, benzoxazoline, imidazoline, benzimidazoline, pyrazoline ou benzopyrazoline, chacun d'eux portant éventuellement un ou deux substituants identiques ou différents choisis parmi les groupes alkyle en C₁ à C₈ eux-mêmes éventuellement substitués par des groupes hydroxy, des halogènes, des groupes cyano, alcoxy en C₁ à C₄, carboxyle, di(alkyle en C₁ à C₄)amino, (alcoxy en C₁ à C₄)carbonyle ou alkylsulfonyle en C₁ à C₄, les groupes alcényle en C₂ à C₄ ou les groupes phényl-alkyle en C₁ à C₂ éventuellement substitués par des halogènes, des groupes cyano, alkyle en C₁ à C₄ ou alcoxy en C₁ à C₄, et
B représente le groupe de formule 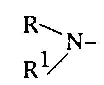
dans laquelle
R représente l'hydrogène, un groupe alkyle cn C₁ à C₈ éventuellement substitué par des groupes hydroxy, des halogènes, des groupes cyano, alcoxy en C₁ à C₄, carboxyle, aminocarbonyle ou (alcoxy en C₁ à C₄)carbonyle, un groupe alcényle en C₂-C₄, un groupe cyclohexyle éventuellement substitué par des groupes alkyle en C₁ à C₄, un groupe phényle, benzyle ou phényléthyle éventuellement substitué par des halogènes, des groupes alkyle en C₁ à C₄ ou alcoxy en C₁ à C₄, un groupe (alkyle en C₁ à C₄)oxycarbonyle, mono- ou di-(alkyle en C₁ à C₄)aminocarbonyle, aminocarbonyle, mono- ou di-(alkyle en C₁ à C₄)aminosulfonyle, amino, alkylamino en C₁ à C₄, di(alkyle en C₁ à C₄)amino, un groupe phénylamino ou benzylamino éventuellement substitué par des groupes alkyle en C₁ à C₄ ou alcoxy en C₁ à C₄ et
R¹ représente l'hydrogène, un groupe alkyle en C₁ à C₈ éventuellement substitué par des groupes hydroxy, des halogènes, des groupes cyano, alcoxy en C₁ à C₄, carboxyle, aminocarbonyle ou (alcoxy en C₁ à C₄)carbonyle, un groupe alcényle en C₂-C₄ ou un groupe phényle, benzyle ou phényléthyle éventuellement substitué par des halogènes, des groupes alkyle en C₁ à C₄ ou alcoxy en C₁ à C₄.

6. Procédé de préparation des colorants de la revendication 4, caractérisé en ce que l'on fait réagir un composé de formule (IV) 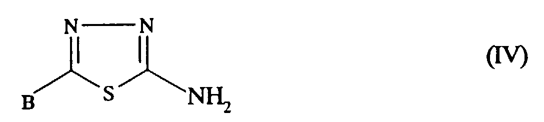 dans laquelle
B a les significations indiquées dans la revendication 4,
et un composé de formule (V)
H-Z¹ (V)
dans laquelle
Z¹ représente un groupe de formule 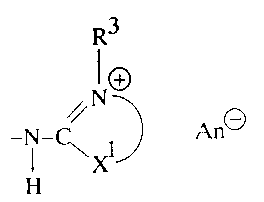
dans laquelle
R³ représente l'hydrogène, un groupe alkyle, alcényle ou aralkyle,
An^{⊖} représente un anion et
X¹ représente les chaînons manquants d'un hétérocycle insaturé à 5 chaînons, éventuellement substitué et éventuellement condensé, qui contient 1 à 3 hétéroatomes choisis parmi l'oxygène, le soufre et l'azote, et qui est quaternisé,
en milieu aqueux, avec une substance libérant de l'acide nitreux, en présence de CO₂ sous une pression de 5 à 100 bar et à une température de 0 à 125°C.

7. Procédé selon la revendication 6, caractérisé en ce que la substance libérant l'acide nitreux est un nitrite minéral ou organique.

8. Procédé pour la teinture ou l'impression de matières naturelles et synthétiques, en particulier de fibres en polyacrylonitrile, en polyesters et polyamides à modification acide, caractérisé en ce que l'on utilise les colorants de la revendication 1.

9. Procédé pour la teinture ou l'impression de matières naturelles et synthétiques, en particulier de fibres et tissus en matières aptes à la teinture par des colorants non ioniques, caractérisé en ce que l'on utilise des colorants de la revendication 4.

10. Procédé pour colorer des résines synthétiques dans la masse, caractérisé en ce que l'on utilise des colorants de la revendication 4.

11. Procédé de préparation des colorants de triazatriméthine selon la revendication 1, caractérisé en ce que l'on fait réagir des bases colorées de formule (II) 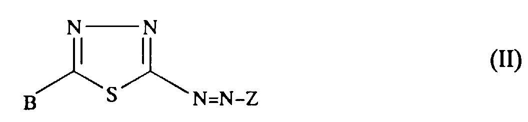 dans laquelle
R et Z ont les significations indiquées dans la revendication 1, avec un agent alkylant choisi parmi les halogénures d'alkyle, les accepteurs de Michael ou les époxydes.
